# EUROPEAN PATENT APPLICATION

(11) **EP 3 985 087 A1**
(43) Date of publication of application: **20.04.2022**
(21) Application number: 20843230.2
(22) Date of filing: 17.07.2020
(51) Int. Cl.: C11B 9/00, A61Q 13/00, C07D 493/08, A23L 27/20, A23L 27/29, A61K 8/49

(54) **FLAVOR COMPOSITION**

(30) Priority: 19.07.2019 JP 2019133854
(71) Applicant: Takasago International Corporation, Tokyo 144-8721 (JP)
(72) Inventor: MURATA, Ariaki, Hiratsuka-shi, Kanagawa 254-0073 (JP); KUSANO, Yumi, Hiratsuka-shi, Kanagawa 254-0073 (JP); TOMIYAMA, Kenichi, Hiratsuka-shi, Kanagawa 254-0073 (JP); KURABE, Aki, Hiratsuka-shi, Kanagawa 254-0073 (JP); TAIRA, Kazuya, Hiratsuka-shi, Kanagawa 254-0073 (JP); NAKAYAMA, Yuji, Hiratsuka-shi, Kanagawa 254-0073 (JP); NAKAZAKI, Atsuo, Nagoya-shi, Aichi 464-8601 (JP)
(74) Representative: Hoffmann Eitle
(86) International application number: PCT/JP2020/027917
(87) International publication number: WO 2021/015135

(57) **Abstract**

The purpose of the present invention is to provide a flavor composition capable of enhancing citrus-like aroma and flavor. The present invention pertains to a flavor composition containing 6a,7-dimethylhexahydro-2H-2,5-methanocyclopenta[b]furan.

## Description

### TECHNICAL FIELD

The present invention relates to a flavor composition capable of imparting or enhancing a citrus-like aroma and flavor, and the use of the flavor composition.

### BACKGROUND ART

Citrus fruits are familiar to people with a refreshing aroma and flavor. Flesh of citrus fruits is eaten, and fruit juice thereof is often used for flavoring of foods, and essential oils and the like obtained from citruses are often used for imparting a citrus-based aroma and flavor to foods and the like.

In order to enhance the citrus-like aroma and flavor, a method of enhancing aroma and flavor by adding various compounds instead of essential oils is also known.

Non-Patent Literature 1 describes a part of isomers of 6a,7-dimethylhexahydro-2H-2,5-methanocyclopenta [b] furan (compound (2) described later).

### CITATION LIST

### NON-PATENT LITERATURE

Non-Patent Literature 1: H. SZALKOWSKA-PAGOWSKA, Polish J. of Chem. 1985, 59 (5-6), 531.

### SUMMARY OF INVENTION

### TECHNICAL PROBLEM

However, Non-Patent Literature 1 does not state that the compound has an odor quality.

An object of the present invention is to provide a flavor composition capable of enhancing a citrus-like aroma and flavor, and to provide various products with the citrus-like aroma and flavor enhanced by using the flavor composition.

### SOLUTION TO PROBLEM

As a result of intensive study to achieve the above objects, the present inventors have found that 6a,7-dimethylhexahydro-2H-2,5-methanocyclopenta [b] furan, which has never been used as a flavor component, can be used to enhance a citrus-like aroma and flavor, leading to the completion of the present invention.

That is, the present invention encompasses the following subject matters [1] to [5].
[1] A flavor composition comprising 6a,7-dimethylhexahydro-2H-2,5-methanocyclopenta [b] furan.
[2] The flavor composition according to [1], which is used for foods or beverages.
[3] A compound represented by the following formula (1). 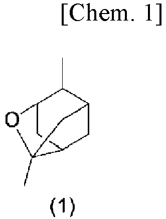
[4] A food or beverage, oral composition, tobacco product, or consumer product, comprising the flavor composition according to [1] or [2] or the compound according to [3].
[5] A method for producing a food or beverage, oral composition, tobacco product, or consumer product, the method comprising adding the flavor composition according to [1] or [2] or the compound according to [3].

### ADVANTAGEOUS EFFECTS OF INVENTION

According to the flavor composition of the present invention, it is possible to impart a citrus-like aroma and flavor to various products or enhance the citrus-like aroma and flavor of various products.

### DESCRIPTION OF EMBODIMENTS

Hereinafter, the present invention will be described in detail.

In the present invention, the compound represented by formula (1) is also referred to as compound (1).

6a,7-dimethylhexahydro-2H-2,5-methanocyclopenta [b] furan in the present invention (hereinafter also referred to as the compound of the present invention) has a structure represented by the following formula (1) or (2). The compound (1) and the compound (2) are in an isomer relationship. It has been found in the present invention that both of the compound (1) and the compound (2) have a citrus-like aroma.

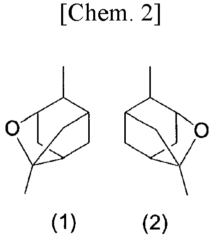

Furthermore, the compounds (1) and (2) have isomers represented by the following formulae (1a), (1b), (2a), and (2b), respectively. That is, 6a,7-dimethylhexahydro-2H-2,5-methanocyclopenta [b] furan has four kinds of isomers.

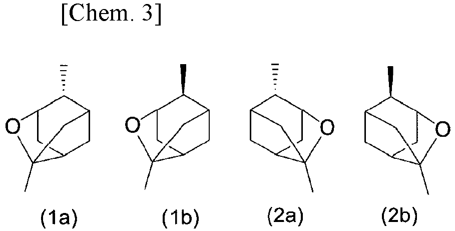

Compound (1a): (2R, 3aR, 5R, 6aR, 7R)-isomer
Compound (1b): (2R, 3aR, 5R, 6aR, 7S)-isomer
Compound (2a): (2S, 3aS, 5S, 6aS, 7S)-isomer
Compound (2b): (2S, 3aS, 5S, 6aS, 7R)-isomer

Regarding a method for producing 6a,7-dimethylhexahydro-2H-2,5-methanocyclopenta [b] furan, a method for synthesizing the compound (1a) and the compound (1b) as an example.

First, R,R-carveol is prepared as a starting raw material, mercury acetate is added to the starting raw material to obtain a mercury-containing bicyclic compound as an intermediate, and NaBH₄ is further added thereto to perform reductive mercury removal, thereby obtaining a mixture of the compound (1a) and the compound (1b).

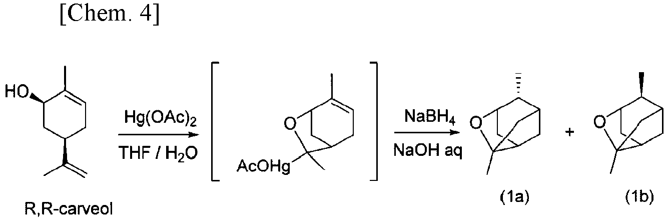

The compound (2a) and the compound (2b) can be synthesized in the same manner as in the synthesis of the compound (1a) and the compound (1b), except that R,R-carveol, which is a starting raw material for the synthesis of the compound (1a) and the compound (1b), is replaced with S,S-carveol.

The flavor composition of the present invention contains 6a,7-dimethylhexahydro-2H-2,5-methanocyclopenta [b] furan.

The content of the compound of the present invention in the flavor composition is preferably 0.00001 to 10,000 ppm, more preferably 0.0001 to 1,000 ppm, and still more preferably 0.001 to 100 ppm, based on the weight of the flavor composition.

All of the four kinds of isomers of 6a,7-dimethylhexahydro-2H-2,5-methanocyclopenta [b] furan are useful as flavor components, and any isomer or isomeric mixture can be used.

Among these, an isomer and an isomeric mixture with a high ratio of the compound (1) are preferred. The ratio of the compound (1) to the compound (2) in the flavor composition is preferably 100:0 to 60:40, and more preferably 100:0 to 80:20.

The compound of the present invention and the flavor composition of the present invention can be directly blended in trace amounts with foods or beverages, oral compositions, tobacco products, and consumer products to impart or enhance a citrus-like aroma and flavor.

As the food or beverage, specific examples thereof include: beverages such as carbonated beverages, soft drinks, fruit juice drinks, milk beverages, lactic acid bacteria beverages, energy drinks, soy milk, and tea drinks; alcoholic drinks such as shochu mixed with soda water, cocktail drinks, sparkling liquor, fruit wine, and alcoholic drinks for medical purposes; desserts such as ice cream, ice milk, lacto-ice, sherbet, yogurt, pudding, jelly, and daily dessert; confectioneries such as caramel, candy, tablet, cracker, biscuit, cookie, pie, chocolate, snack, and chewing gum; soups such as Japanese soup, and Western soup; jams; flavor seasonings; various instant beverages; and various instant foods. Among these, beverages or alcoholic drinks are preferred.

The amount of the compound of the present invention added to a food or beveragemay be in a concentration range of 0.00001 to 10,000 ppb, preferably 0.0001 to 1,000 ppb, and more preferably 0.001 to 100 ppb, with respect to the weight of the food or beverage.

The amount of the flavor composition added to the food or beverage varies depending on the kind and form of the product, and is generally in a concentration range of 0.001 to 10 mass%, preferably 0.01 to 5 mass%, based on the mass of the food or beverage before the flavor composition is added.

As the oral compositions, examples thereof include toothpastes, oral rinses, mouthwashes, troches, chewing gums, and the like.

The amount of the compound of the present invention added to the oral composition may be in a concentration range of 0.0001 to 100,000 ppb, preferably 0.001 to 10,000 ppb, and more preferably 0.01 to 1,000 ppb, with respect to the weight of the oral composition.

The amount of the flavor composition added to the oral composition varies depending on the kind and form of the product, and is usually preferably 0.01 to 5 mass%, more preferably 0.1 to 3 mass%, based on the total mass of the oral composition.

As the tobacco products, examples thereof include cigarettes, electronic cigarettes, and the like.

The amount of the compound of the present invention added to the tobacco product may be in a concentration range of 0.0001 to 100 ppm, preferably 0.001 to 10 ppm, and more preferably 0.01 to 1 ppm, with respect to the weight of the tobacco product.

The amount of the flavor composition added to the tobacco product varies depending on the kind and form of the product, and may be, for example, preferably 0.00001 to 90 mass%, and more preferably 0.0001 to 50 mass%, with respect to the total mass of the leaves of tobaccos.

As the consumer product, specific examples thereof include:
fragrance products such as perfume, eau de parfum, eau de toilette, and eau de cologne;
foundation cosmetics such as facial wash creams, vanishing creams, cleansing creams, cold creams, massage creams, milky lotions, skin lotions, beauty lotions, facial packs, and makeup removers;
finishing cosmetics such as foundations, face powders, solid face powders, talcum powders, rouges, lip balms, cheek rouges, eye liners, mascara, eye shadows, eyebrow pencils, eye packs, nail enamels, and enamel removers;
hair cosmetics such as pomade, brilliantine, hair set lotions, hair sticks, hair solids, hair oils, hair treatments, hair creams, hair tonics, hair liquids, hair sprays, bandolines, revitalizing hair tonics, and hair dyes;
suntan cosmetics such as suntan products and sun-screen products;
medicated cosmetics such as antiperspirants, after-shaving lotions or gels, permanent wave agents, medicated soaps, medicated shampoos, and medicated skin cosmetics;
hair care products such as shampoos, rinses, rinse-in-shampoos, conditioners, treatments, and hair packs;
soap such as toilet soaps, bath soaps, perfume soaps, transparent soaps, and synthetic soaps;
body washers such as body soaps, body shampoos, hand soaps, and face creams;
bath agents such as bathing agents (such as bath salts, bath tablets, and bath liquids), foam bath (such as bubble bath), bath oils (such as bath perfumes, and bath capsules), milk-baths, bath jelly, and bath cubes;
detergents such as heavy detergents for clothes, light detergents for clothes, liquid detergents, washing soaps, compact detergents, and powder soaps;
soft finishing agents such as softener and furniture care;
cleaners such as cleansers, house cleaners, toilet cleaners, bath cleaners, glass cleaners, mildew removers, and cleaners for drainpipe use;
kitchen cleaners such as kitchen soaps, kitchen synthetic soaps, and tableware cleaners;
bleaching agents such as oxidation type bleaching agents (such as chlorine type bleaching agents, and oxygen type bleaching agents), reduction type bleaching agents (such as sulfur type bleaching agents), and optical bleaching agents;
aerosol agents such as spray type ones, and powder sprays;
deodorants or aromatics such as solid type ones, gel type ones, and liquid type ones (aqueous or oily);
daily necessities and household goods such as tissue papers, and toilet papers;
quasi-drugs such as liquid bath additives, mouthwashes, and repellents (such as mist spray type ones and aqueous liquid type ones); and
pharmaceuticals such as medicinal cosmetics and medicinal lotions.

The amount of the compound of the present invention added to the consumer product may be in a concentration range of 0.0001 to 100,000 ppb, preferably 0.001 to 10,000 ppb, and more preferably 0.01 to 1,000 ppb, with respect to the weight of the consumer product.

The amount of the flavor composition added to the consumer product varies depending on the kind and form of the product, and may be usually 0.001 to 90 mass%, preferably 0.01 to 50 mass%, and more preferably 0.1 to 25 mass%, respectively, based on the mass of the consumer product before the flavor composition is added.

In addition, the compound or flavor composition of the present invention may be mixed with other flavor components, and the mixture is added to each of the above products and compositions to impart or enhance the citrus-like aroma and flavor. As the other flavor components, various synthetic flavors, natural flavors, natural essential oils, plant extracts, or the like may be mentioned, and examples thereof include natural essential oils, natural flavors, and synthetic flavors as described in "Japan Patent Office Gazette, Known Customary Technologies (Flavor), Part II: Food Flavor, published on January 14, 2000, p.88 to 131".

Preferred examples of the citrus of the citrus-like aroma and flavor imparted or enhanced in the present invention include grapefruit, orange, lemon, lime, yuzu, blood orange, bitter orange, tangerine, mandarin orange, satsuma mandarin orange, amanatsu, natsumikan, hassaku, pomelo, kabosu, sudachi, hebesu, bergamot, and sweetie (oroblanco), but the citrus is not limited to those described herein.

### [Example]

Hereinafter, the present invention will be described in more detail with reference to Examples, but the present invention is not limited to these Examples.

The analytical instruments used in the examples are as follows.
NMR measurement device: AVANCE III 500 (manufactured by BRUKER BIO SPIN K.K.)
Gas chromatograph-mass spectrometer: GCMS-QP2010 (manufactured by Shimadzu Corporation)
Gas chromatograph-orbitrap mass spectrometer: TRACE1310 (GC) + Exactive GC (mass spectrometer) (manufactured by Thermo Fisher Scientific Inc.)
High performance liquid chromatograph
   Pump: LC-20A (manufactured by Shimadzu Corporation)
   Detector: SPD-M20A (manufactured by GL Sciences Inc.)
   Separation column: YMC-Triart C18 ExRS (manufactured by YMC Co., Ltd.)

### (Example 1) (Synthesis of (2R,3aR,5R,6aR,7R)-6a,7-dimethylhexahydro-2H-2,5-methanocyclopenta [b] furan (compound (1a)) and (2R,3aR,5R,6aR,7S)-6a,7-dimethylhexahydro-2H-2,5-methanocyclopenta [b] furan (compound (1b))

30.4 g of (R,R)-carveol was made into a 200 mL THF solution. 61.2 g of mercury acetate was added to the THF solution and was allowed to react at 20°C for 9 days to obtain an intermediate mercury-containing bicyclic compound. Thereafter, 50 mL of a 3M aqueous sodium hydroxide solution and 11.4 g of NaBH₄ were added and were allowed to react at 15°C for 3 hours to perform reductive mercury removal, thereby obtaining a mixture of (2R,3aR,5R,6aR,7R)-6a,7-dimethylhexahydro-2H-2,5-methanocyclopenta [b] furan and (2R,3aR,5R,6aR,7S)-6a,7-dimethylhexahydro-2H-2,5-methanocyclopenta [b] furan.

Subsequently, purification was performed by high performance liquid chromatography (eluent; water:acetonitrile = 70:30 (volume ratio)) to obtain the target (2R,3aR,5R,6aR,7R)-6a,7-dimethylhexahydro-2H-2,5-methanocyclopenta [b] furan and (2R,3aR,5R,6aR,7S)-6a,7-dimethylhexahydro-2H-2,5-methanocyclopenta [b] furan (GC purity: 100%).

### <Physical Data of (2R,3aR,5R,6aR,7R)-6a,7-Dimethylhexahydro-2H-2,5-methanocyclopenta [b] furan>

¹H NMR (500 MHz, CDCl3) δ:
4.02 (t, J = 3.60 Hz, 1H), 2.10 (t, J = 6.65 Hz, 1H), 2.03 (m, 1H), 1.96 (m, 1H), 1.90 (d, J = 11.06 Hz, 1H), 1.88 (dd, J = 11.15 Hz, 3.30 Hz, 1H), 1.78 (m, 1H), 1.77 (m, 1H), 1.48 (m, 1H), 1.47 (dd, J = 11.06 Hz, 4.00 Hz, 1H), 1.38 (s, 3H), 0.89 (d, J = 7.21 Hz, 3H),
¹³C NMR (125 MHz, CDCl3) δ:
   85.10, 81.54, 47.31, 44.87, 42.10, 38.17, 37.04, 36.52, 23.14, 15.66
   HRMS(CI+): calcd C10H17O([M+H]+) 153.1274; found, 153.1274

### <Physical Data of (2R,3aR,5R,6aR,7S)-6a,7-Dimethylhexahydro-2H-2,5-methanocyclopenta [b] furan>

¹H NMR (500 MHz, CDCl3) δ:
3.96 (d, J = 4.55 Hz, 1H), 2.17 (t, J = 6.60 Hz, 1H), 2.08 (dd, J = 11.75 Hz, 3.50 Hz, 1H), 2.06 (m, 1H), 1.96 (m, 1H), 1.73 (m, 1H), 1.71 (d, J = 10.55 Hz, 1H), 1.61 (dd, J = 11.40 Hz, 3.00 Hz, 1H), 1.57 (dd, J = 7.00 Hz, 2.40 Hz, 1H), 1.35 (s, 3H), 1.24 (dd, J = 11.75 Hz, 2.80 Hz, 1H), 1.05 (d, J = 7.00 Hz, 3H), ¹³C NMR (125 MHz, CDCl3) δ:
86.47, 81.78, 43.89, 42.52, 42.33, 42.24, 42.10, 36.36, 23.39, 17.31
HRMS(CI+): calcd C10H17O([M+H]+) 153.1274; found, 153.1274

### (Example 2) (Synthesis of (2S,3aS,5S,6aS,7S)-6a,7-dimethylhexahydro-2H-2,5-methanocyclopenta [b] furan (compound (2a)) and (2S,3aS,5S,6aS,7R)-6a,7-dimethylhexahydro-2H-2,5-methanocyclopenta [b] furan (compound (2b))

A mixture of (2S,3aS,5S,6aS,7S)-6a,7-dimethylhexahydro-2H-2,5-methanocyclopenta [b] furan and (2S,3aS,5S,6aS,7R)-6a,7-dimethylhexahydro-2H-2,5-methanocyclopenta [b] furan was obtained in the same manner as in Example 1 except that (R,R)-carveol was changed to (S, S)-carveol.

Subsequently, purification was performed by high performance liquid chromatography (eluent; water:acetonitrile = 70:30 (volume ratio)) to obtain the target (2S,3aS,5S,6aS,7S)-6a,7-dimethylhexahydro-2H-2,5-methanocyclopenta [b] furan and (2S,3aS,5S,6aS,7R)-6a,7-dimethylhexahydro-2H-2,5-methanocyclopenta [b] furan (GC purity: 100%).

### <Physical Data of (2S,3aS,5S,6aS,7S)-6a,7-Dimethylhexahydro-2H-2,5-methanocyclopenta [b] furan>

¹H NMR (500 MHz, CDCl3) δ:
The compound is predicted to be the same as the compound (1a).

¹³C NMR (125 MHz, CDCl3) δ:
The compound is predicted to be the same as the compound (1a).

HRMS(CI+): calcd C10H17O([M+H]+) 153.1274; found, 153.1274

### <Physical Data of (2S,3aS,5S,6aS,7R)-6a,7-Dimethylhexahydro-2H-2,5-methanocyclopenta [b] furan>

¹H NMR (500 MHz, CDCl3) δ:

The compound is predicted to be the same as the compound (1b).

¹³C NMR (125 MHz, CDCl3) δ:
The compound is predicted to be the same as the compound (1b).

HRMS(CI+): calcd C10H17O([M+H]+) 153.1274; found, 153.1274

### (Example 1-1) Production of Orange Flavor Composition

After the production in Example 1, the isolated (2R,3aR,5R,6aR,7R)-6a,7-dimethylhexahydro-2H-2,5-methanocyclopenta [b] furan (compound (1a)) was used to prepare an orange flavor composition (A) based on the following formulation. For comparison, an orange flavor composition (B) was prepared based on the following formulation.

**[Table 1]**

| (Component) | Flavor composition (A) (parts by weight) | Flavor composition (B) (parts by weight) |
|---|---|---|
| (2R,3aR,5R,6aR,7R)-6a,7-dimethylhexahydro- 2H-2,5-methanocyclopenta [b] furan (compound (1a)) isolated after production in Example 1 | 0.01 | - |
| Orange oil (cold pressed) | 50.0 | 50.0 |
| Ethanol | Residue | Residue |
| Total | 1000.0 | 1000.0 |

Each of the orange flavor compositions (A) and (B) prepared according to the above formulation was added to water at a concentration of 0.1%, and a sensory test was conducted by 11 expert panelists. As a result, all of the panelists commented that the flavor composition (A) was significantly superior to the flavor composition (B) in terms of impartation of the real fruit juice feeling, freshness, and fresh feeling. The same was true for the case where (2R,3aR,5R,6aR,7R)-6a,7-dimethylhexahydro-2H-2,5-methanocyclopenta [b] furan in the flavor composition (A) was replaced with (2R,3aR,5R,6aR,7S)-6a,7-dimethylhexahydro-2H-2,5-methanocyclopenta [b] furan (compound (1b)).

### (Example 2-1) Production of Orange Flavor Composition

After the production in Example 2, the isolated (2S,3aS,5S,6aS,7S)-6a,7-dimethylhexahydro-2H-2,5-methanocyclopenta [b] furan (compound (2a)) was used to prepare an orange flavor composition (C) based on the following formulation. For comparison, an orange flavor composition (D) was prepared based on the following formulation.

**[Table 2]**

| (Component) | Flavor composition (C) (parts by weight) | Flavor composition (D) (parts by weight) |
|---|---|---|
| (2S,3aS,5S,6aS,7S)-6a,7-dimethylhexahydro- 2H-2,5-methanocyclopenta [b] furan (compound (2a)) isolated after production in Example 2 | 0.01 | - |
| Orange oil (cold pressed) | 50.0 | 50.0 |
| Ethanol | Residue | Residue |
| Total | 1000.0 | 1000.0 |

Each of the orange flavor compositions (C) and (D) prepared according to the above formulation was added to water at a concentration of 0.1%, and a sensory test was conducted by 11 expert panelists. As a result, all of the panelists commented that the flavor composition (C) was significantly superior to the flavor composition (D) in terms of enhancement of the real green feeling and fresh feeling. The same was true for the case where (2S,3aS,5S,6aS,7S)-6a,7-dimethylhexahydro-2H-2,5-methanocyclopenta [b] furan in the flavor composition (C) was replaced with (2S,3aS,5S,6aS,7R)-6a,7-dimethylhexahydro-2H-2,5-methanocyclopenta [b] furan (compound (2b)).

### (Example 3) Production of Orange Flavor Composition

The 6a,7-dimethylhexahydro-2H-2,5-methanocyclopenta [b] furan produced in Example 1 (i.e., a mixture of the compound (1a) and the compound (1b).The same applies to the subsequent examples.) was used to prepare an orange flavor composition (1) based on the following formulation. For comparison, an orange flavor composition (2) was prepared based on the following formulation.

**[Table 3]**

| (Component) | Flavor composition (1) (parts by weight) | Flavor composition (2) (parts by weight) |
|---|---|---|
| 6a,7-dimethylhexahydro-2H-2,5 -methanocyclopenta [b] furan in Example 1 | 0.01 | - |
| Orange oil (cold pressed) | 50.0 | 50.0 |
| Ethanol | Residue | Residue |
| Total | 1000.0 | 1000.0 |

Each of the orange flavor compositions (1) and (2) prepared according to the above formulation was added to water at a concentration of 0.1%, and a sensory test was conducted by 11 expert panelists. As a result, all of the panelists commented that the flavor composition (1) was significantly superior to the flavor composition (2) in terms of impartation of the real fruit juice feeling, freshness, and fresh feeling. The same was true for the case where no matter which of the four kinds of isomers 6a,7-dimethylhexahydro-2H-2,5-methanocyclopenta [b] furan in the flavor composition (1) was, it was replaced with an isomeric mixture (i.e., a mixture of the compound (2a) and the compound (2b)).

### (Example 4) Production of Orange Flavor Composition

An orange flavor composition (3) was prepared based on the following formulation using 6a,7-dimethylhexahydro-2H-2,5-methanocyclopenta [b] furan produced in Example 1. For comparison, an orange flavor composition (4) was prepared based on the following formulation.

**[Table 4]**

| (Component) | Flavor composition (3) (parts by weight) | Flavor composition (4) (parts by weight) |
|---|---|---|
| 6a,7-dimethylhexahydro-2H-2,5-methanocyclopenta [b] furan in Example 1 | 0.01 | - |
| Ethyl butyrate | 0.5 | 0.5 |
| Octanal | 1.0 | 1.0 |
| Nonanal | 0.1 | 0.1 |
| Decanal | 0.5 | 0.5 |
| Linalool | 2.0 | 2.0 |
| Ethanol | Residue | Residue |
| Total | 1000.0 | 1000.0 |

Each of the orange flavor compositions (3) and (4) prepared according to the above formulation was added to water at a concentration of 0.1%, and a sensory test was conducted by 11 expert panelists. As a result, all of the panelists commented that the flavor composition (3) was significantly superior to the flavor composition (4) in terms of impartation of the real fruit juice feeling, freshness, and fresh feeling. The same was true for the case where no matter which of the four kinds of isomers 6a,7-dimethylhexahydro-2H-2,5-methanocyclopenta [b] furan in the flavor composition (3) was, it was replaced with an isomeric mixture.

### (Example 5) Production of Lemon Flavor Composition

A lemon flavor composition (5) was prepared based on the following formulation using 6a,7-dimethylhexahydro-2H-2,5-methanocyclopenta [b] furan produced in Example 1. For comparison, a lemon flavor composition (6) was prepared based on the following formulation.

**[Table 5]**

| (Component) | Flavor composition (5) (parts by weight) | Flavor composition (6) (parts by weight) |
|---|---|---|
| 6a,7-dimethylhexahydro-2H-2,5 -methanocyclopenta [b] furan in Example 1 | 0.01 | - |
| Lemon oil (cold pressed) | 50.0 | 50.0 |
| Ethanol | residue | residue |
| Total | 1000.0 | 1000.0 |

Each of the lemon flavor compositions (5) and (6) prepared according to the above formulation was added to water at a concentration of 0.1%, and a sensory test was conducted by 11 expert panelists. As a result, all of the panelists commented that the flavor composition (5) was significantly superior to the flavor composition (6) in terms of impartation of fresh feeling and fresh and sweet aroma. The same was true for the case where no matter which of the four kinds of isomers 6a,7-dimethylhexahydro-2H-2,5-methanocyclopenta [b] furan in the flavor composition (5) was, it was replaced with an isomeric mixture.

### (Example 6) Production of Lemon Flavor Composition

A lemon flavor composition (7) was prepared based on the following formulation using 6a,7-dimethylhexahydro-2H-2,5-methanocyclopenta [b] furan produced in Example 1. For comparison, a lemon flavor composition (8) was prepared based on the following formulation.

**[Table 6]**

| (Component) | Flavor composition (7) (parts by weight) | Flavor composition (8) (parts by weight) |
|---|---|---|
| 6a,7-dimethylhexahydro-2H-2,5 -methanocyclopenta [b] furan in Example 1 | 0.01 | - |
| Citral | 3.0 | 3.0 |
| α-terpineol | 1.0 | 1.0 |
| Geraniol | 0.5 | 0.5 |
| Geranyl acetate | 0.5 | 0.5 |
| Neryl acetate | 0.5 | 0.5 |
| Ethanol | Residue | Residue |
| Total | 1000.0 | 1000.0 |

Each of the lemon flavor compositions (7) and (8) prepared according to the above formulation was added to water at a concentration of 0.1%, and a sensory test was conducted by 11 expert panelists. As a result, all of the panelists commented that the flavor composition (7) was significantly superior to the flavor composition (8) in terms of impartation of fresh feeling and fresh and sweet aroma. The same was true for the case where no matter which of the four kinds of isomers 6a,7-dimethylhexahydro-2H-2,5-methanocyclopenta [b] furan in the flavor composition (7) was, it was replaced with an isomeric mixture.

### (Example 7) Production of Grapefruit Flavor Composition

A grapefruit flavor composition (9) was prepared based on the following formulation using 6a,7-dimethylhexahydro-2H-2,5-methanocyclopenta [b] furan produced in Example 1. For comparison, a grapefruit flavor composition (10) was prepared based on the following formulation.

**[Table 7]**

| (Component) | Flavor composition (9) (parts by weight) | Flavor composition (10) (parts by weight) |
|---|---|---|
| 6a,7-dimethylhexahydro-2H-2,5-methanocyclopenta [b] furan in Example 1 | 0.01 | - |
| Grapefruit oil (cold pressed) | 50.0 | 50.0 |
| Ethanol | Residue | Residue |
| Total | 1000.0 | 1000.0 |

Each of the grapefruit flavor compositions (9) and (10) prepared according to the above formulation was added to water at a concentration of 0.1%, and a sensory test was conducted by 11 expert panelists. As a result, all of the panelists commented that the flavor composition (9) was significantly superior to the flavor composition (10) in terms of impartation of the real fruit juice feeling, freshness, and fresh feeling. The same was true for the case where no matter which of the four kinds of isomers 6a,7-dimethylhexahydro-2H-2,5-methanocyclopenta [b] furan in the flavor composition (9) was, it was replaced with an isomeric mixture.

### (Example 8) Production of Grapefruit Flavor Composition

A grapefruit flavor composition (11) was prepared based on the following formulation using 6a,7-dimethylhexahydro-2H-2,5-methanocyclopenta [b] furan produced in Example 1. For comparison, a grapefruit flavor composition (12) was prepared based on the following formulation.

**[Table 8]**

| (Component) | Flavor composition (11) (parts by weight) | Flavor composition (12) (parts by weight) |
|---|---|---|
| 6a,7-dimethylhexahydro-2H-2,5-methanocyclopenta [b] furan in Example 1 | 0.01 | - |
| Nootkatone | 2.0 | 2.0 |
| Octanal | 0.3 | 0.3 |
| Decanal | 0.2 | 0.2 |
| Dodecanal | 0.1 | 0.1 |
| Linalool | 0.8 | 0.8 |
| Cis-3-hexenol | 0.5 | 0.5 |
| Ethanol | Residue | Residue |
| Total | 1000.0 | 1000.0 |

Each of the grapefruit flavor compositions (11) and (12) prepared according to the above formulation was added to water at a concentration of 0.1%, and a sensory test was conducted by 11 expert panelists. As a result, all of the panelists commented that the flavor composition (11) was significantly superior to the flavor composition (12) in terms of impartation of the real fruit juice feeling, freshness, and fresh feeling. The same was true for the case where no matter which of the four kinds of isomers 6a,7-dimethylhexahydro-2H-2,5-methanocyclopenta [b] furan in the flavor composition (11) was, it was replaced with an isomeric mixture.

### (Example 9) Production of Lime Flavor Composition

A lime flavor composition (13) was prepared based on the following formulation using 6a,7-dimethylhexahydro-2H-2,5-methanocyclopenta [b] furan produced in Example 1. For comparison, a lime flavor composition (14) was prepared based on the following formulation.

**[Table 9]**

| (Component) | Flavor composition (13) (parts by weight) | Flavor composition (14) (parts by weight) |
|---|---|---|
| 6a,7-dimethylhexahydro-2H-2,5-methanocyclopenta [b] furan in Example 1 | 0.01 | - |
| Lime oil (distilled) | 50.0 | 50.0 |
| Ethanol | Residue | Residue |
| Total | 1000.0 | 1000.0 |

Each of the lime flavor compositions (13) and (14) prepared according to the above formulation was added to water at a concentration of 0.1%, and a sensory test was conducted by 11 expert panelists. As a result, all of the panelists commented that the flavor composition (13) was significantly superior to the flavor composition (14) in terms of impartation of fresh and sweet aroma. The same was true for the case where no matter which of the four kinds of isomers 6a,7-dimethylhexahydro-2H-2,5-methanocyclopenta [b] furan in the flavor composition (13) was, it was replaced with an isomeric mixture.

### (Example 10) Production of Lime Flavor Composition

A lime flavor composition (15) was prepared based on the following formulation using 6a,7-dimethylhexahydro-2H-2,5-methanocyclopenta [b] furan produced in Example 1. For comparison, a lime flavor composition (16) was prepared based on the following formulation.

**[Table 10]**

| (Component) | Flavor composition (15) (parts by weight) | Flavor composition (16) (parts by weight) |
|---|---|---|
| 6a,7-dimethylhexahydro-2H-2,5-methanocyclopenta [b] furan in Example 1 | 0.01 | - |
| Citral | 1.0 | 1.0 |
| Terpinen-4-ol | 1.0 | 1.0 |
| 1,8-cineole | 2.0 | 2.0 |
| Geranyl acetate | 1.0 | 1.0 |
| Fenchol | 0.5 | 0.5 |
| Ethanol | Residue | Residue |
| Total | 1000.0 | 1000.0 |

Each of the lime flavor compositions (15) and (16) prepared according to the above formulation was added to water at a concentration of 0.1%, and a sensory test was conducted by 11 expert panelists. As a result, all of the panelists commented that the flavor composition (15) was significantly superior to the flavor composition (16) in terms of impartation of fresh and sweet aroma. The same was true for the case where no matter which of the four kinds of isomers 6a,7-dimethylhexahydro-2H-2,5-methanocyclopenta [b] furan in the flavor composition (17) was, it was replaced with an isomeric mixture.

### (Example 11) Production of Yuzu Flavor Composition

A yuzu flavor composition (17) was prepared based on the following formulation using 6a,7-dimethylhexahydro-2H-2,5-methanocyclopenta [b] furan produced in Example 1. For comparison, a yuzu flavor composition (18) was prepared based on the following formulation.

**[Table 11]**

| (Component) | Flavor composition (17) (parts by weight) | Flavor composition (18) (parts by weight) |
|---|---|---|
| 6a,7-dimethylhexahydro-2H-2,5-methanocyclopenta [b] furan in Example 1 | 0.01 | - |
| Yuzu oil (cold pressed) | 50.0 | 50.0 |
| Ethanol | Residue | Residue |
| Total | 1000.0 | 1000.0 |

Each of the yuzu flavor compositions (17) and (18) prepared according to the above formulation was added to water at a concentration of 0.1%, and a sensory test was conducted by 11 expert panelists. As a result, all of the panelists commented that the flavor composition (17) was significantly superior to the flavor composition (18) in terms of impartation of fresh and sweet aroma. The same was true for the case where no matter which of the four kinds of isomers 6a,7-dimethylhexahydro-2H-2,5-methanocyclopenta [b] furan in the flavor composition (17) was, it was replaced with an isomeric mixture.

### (Example 12) Production of Yuzu Flavor Composition

A yuzu flavor composition (19) was prepared based on the following formulation using 6a,7-dimethylhexahydro-2H-2,5-methanocyclopenta [b] furan produced in Example 1. For comparison, a yuzu flavor composition (20) was prepared based on the following formulation.

**[Table 12]**

| (Component) | Flavor composition (19) (parts by weight) | Flavor composition (20) (parts by weight) |
|---|---|---|
| 6a,7-dimethylhexahydro-2H-2,5-methanocyclopenta [b] furan in Example 1 | 0.01 | - |
| Citronellol | 1.0 | 1.0 |
| Octanal | 1.0 | 1.0 |
| Carvacrol | 1.0 | 1.0 |
| Cis-3-hexenal | 1.0 | 1.0 |
| Perillaldehyde | 1.0 | 1.0 |
| Neryl acetate | 2.0 | 2.0 |
| α-terpineol | 2.0 | 2.0 |
| Thymol | 3.0 | 3.0 |
| α-pinene | 8.0 | 8.0 |
| Linalool | 30.0 | 30.0 |
| Myrcene | 50.0 | 50.0 |
| γ-terpinene | 10.0 | 10.0 |
| Limonene | 300.0 | 300.0 |
| Ethanol | Residue | Residue |
| Total | 1000.0 | 1000.0 |

Each of the yuzu flavor compositions (19) and (20) prepared according to the above formulation was added to water at a concentration of 0.1%, and a sensory test was conducted by 11 expert panelists. As a result, all of the panelists commented that the flavor composition (19) was significantly superior to the flavor composition (20) in terms of impartation of fresh and sweet aroma. The same was true for the case where no matter which of the four kinds of isomers 6a,7-dimethylhexahydro-2H-2,5-methanocyclopenta [b] furan in the flavor composition (19) was, it was replaced with an isomeric mixture.

### (Example 13) Production of Cider Flavor Composition

A cider flavor composition (21) was prepared based on the following formulation using 6a,7-dimethylhexahydro-2H-2,5-methanocyclopenta [b] furan produced in Example 1. For comparison, a cider flavor composition (22) was prepared based on the following formulation.

**[Table 13]**

| (Component) | Flavor composition (21) (parts by weight) | Flavor composition (22) (parts by weight) |
|---|---|---|
| 6a,7-dimethylhexahydro-2H-2,5-methanocyclopenta [b] furan in Example 1 | 0.1 | - |
| Lemon oil (cold pressed) | 10.0 | 10.0 |
| Lime oil (distilled) | 5.0 | 5.0 |
| Orange oil (cold pressed) | 20.0 | 20.0 |
| Isoamyl acetate | 10.0 | 10.0 |
| Ethyl isovalerate | 1.0 | 1.0 |
| Ethyl butyrate | 2.0 | 2.0 |
| Vanillin | 0.005 | 0.005 |
| Ethanol | Residue | Residue |
| Total | 1000.0 | 1000.0 |

Each of the cider flavor compositions (21) and (22) prepared according to the above formulation was added to water at a concentration of 0.1%, and a sensory test was conducted by 11 expert panelists. As a result, all of the panelists commented that the flavor composition (21) was significantly superior to the flavor composition (22) in terms of impartation of citrus-like fresh sweetness and a carbonation feeling. The same was true for the case where no matter which of the four kinds of isomers 6a,7-dimethylhexahydro-2H-2,5-methanocyclopenta [b] furan in the flavor composition (21) was, it was replaced with an isomeric mixture.

### (Example 14) Production of Apple Flavor Composition

An apple flavor composition (23) was prepared based on the following formulation using 6a,7-dimethylhexahydro-2H-2,5-methanocyclopenta [b] furan produced in Example 1. For comparison, an apple flavor composition (24) was prepared based on the following formulation.

**[Table 14]**

| (Component) | Flavor composition (23) (parts by weight) | Flavor composition (24) (parts by weight) |
|---|---|---|
| 6a,7-dimethylhexahydro-2H-2,5-methanocyclopenta [b] furan in Example 1 | 0.1 | - |
| 2-Methylbutyl acetate | 16.0 | 16.0 |
| Hexanol | 12.0 | 12.0 |
| Trans-2-hexenal | 1.5 | 1.5 |
| Isoamyl alcohol | 10.0 | 10.0 |
| Hexanal | 3.0 | 3.0 |
| Hexyl acetate | 6.0 | 6.0 |
| Butyl acetate | 12.0 | 12.0 |
| Ethyl butyrate | 3.0 | 3.0 |
| Ethyl 2-methylbutyrate | 2.0 | 2.0 |
| Acetic acid | 4.0 | 4.0 |
| Damascenone | 0.005 | 0.005 |
| Ethanol | Residue | Residue |
| Total | 1000.0 | 1000.0 |

Each of the apple flavor compositions (23) and (24) prepared according to the above formulation was added to water at a concentration of 0.1%, and a sensory test was conducted by 11 expert panelists. As a result, all of the panelists commented that the flavor composition (23) was significantly superior to the flavor composition (24) in terms of impartation of a natural fruit juice feeling. The same was true for the case where no matter which of the four kinds of isomers 6a,7-dimethylhexahydro-2H-2,5-methanocyclopenta [b] furan in the flavor composition (23) was, it was replaced with an isomeric mixture.

### (Example 15) Production of Grape Flavor Composition

A grape flavor composition (25) was prepared based on the following formulation using 6a,7-dimethylhexahydro-2H-2,5-methanocyclopenta [b] furan produced in Example 1. For comparison, a grape flavor composition (26) was prepared based on the following formulation.

**[Table 15]**

| (Component) | Flavor composition (25) (parts by weight) | Flavor composition (26) (parts by weight) |
|---|---|---|
| 6a,7-dimethylhexahydro-2H-2,5-methanocyclopenta [b] furan in Example 1 | 0.1 | - |
| Ethyl acetate | 30.0 | 30.0 |
| Ethyl butyrate | 20.0 | 20.0 |
| Cis-3-hexenol | 8.0 | 8.0 |
| Methyl anthranilate | 15.0 | 15.0 |
| Ethyl maltol | 20.0 | 20.0 |
| Propionic acid | 15.0 | 15.0 |
| Hexanoic acid | 1.5 | 1.5 |
| Trans-2-hexenal | 2.0 | 2.0 |
| Propyl acetate | 25.0 | 25.0 |
| Ethyl Propionate | 30.0 | 30.0 |
| Linalool | 0.3 | 0.3 |
| Cinnamic alcohol | 0.3 | 0.3 |
| Damascenone | 0.002 | 0.002 |
| Ethanol | Residue | Residue |
| Total | 1000.0 | 1000.0 |

Each of the grape flavor compositions (25) and (26) prepared according to the above formulation was added to water at a concentration of 0.1%, and a sensory test was conducted by 11 expert panelists. As a result, all of the panelists commented that the flavor composition (25) was significantly superior to the flavor composition (26) in terms of impartation of a natural fruit juice feeling. The same was true for the case where no matter which of the four kinds of isomers 6a,7-dimethylhexahydro-2H-2,5-methanocyclopenta [b] furan in the flavor composition (25) was, it was replaced with an isomeric mixture.

### (Example 16) Production of Peach Flavor Composition

A peach flavor composition (27) was prepared based on the following formulation using 6a,7-dimethylhexahydro-2H-2,5-methanocyclopenta [b] furan produced in Example 1. For comparison, a peach flavor composition (28) was prepared based on the following formulation.

**[Table 16]**

| (Component) | Flavor composition (27) (parts by weight) | Flavor composition (28) (parts by weight) |
|---|---|---|
| 6a,7-dimethylhexahydro-2H-2,5-methanocyclopenta [b] furan in Example 1 | 0.1 | - |
| Ethyl acetate | 50.0 | 50.0 |
| Hexanol | 1.0 | 1.0 |
| Benzaldehyde | 0.4 | 0.4 |
| Maltol | 2.0 | 2.0 |
| Hexanal | 0.2 | 0.2 |
| Hexyl acetate | 3.0 | 3.0 |
| γ-undecalactone | 0.3 | 0.3 |
| Ethyl octanoate | 0.05 | 0.05 |
| β-Ionone | 0.002 | 0.002 |
| Linalool | 0.2 | 0.2 |
| Isoamyl acetate | 8.0 | 8.0 |
| Ethyl butyrate | 3.0 | 3.0 |
| Ethanol | Residue | Residue |
| Total | 1000.0 | 1000.0 |

Each of the peach flavor compositions (27) and (28) prepared according to the above formulation was added to water at a concentration of 0.1%, and a sensory test was conducted by 11 expert panelists. As a result, all of the panelists commented that the flavor composition (27) was significantly superior to the flavor composition (28) in terms of impartation of a natural fruit juice feeling. The same was true for the case where no matter which of the four kinds of isomers 6a,7-dimethylhexahydro-2H-2,5-methanocyclopenta [b] furan in the flavor composition (27) was, it was replaced with an isomeric mixture.

### (Example 17) Production of Pineapple Flavor Composition

A pineapple flavor composition (29) was prepared based on the following formulation based on 6a,7-dimethylhexahydro-2H-2,5-methanocyclopenta [b] furan produced in Example 1. For comparison, a pineapple flavor composition (30) was prepared based on the following formulation.

**[Table 17]**

| (Component) | Flavor composition (29) (parts by weight) | Flavor composition (30) (parts by weight) |
|---|---|---|
| 6a,7-dimethylhexahydro-2H-2,5 -methanocyclopenta [b] furan in Example 1 | 0.1 | - |
| Ethyl acetate | 40.0 | 40.0 |
| Allyl hexanoate | 20.0 | 20.0 |
| Ethyl 2-methylbutyrate | 8.0 | 8.0 |
| Ethyl octanoate | 1.0 | 1.0 |
| Ethyl acrylate | 0.02 | 0.02 |
| Ethyl trans-3-hexenoate | 0.04 | 0.04 |
| γ-octalactone | 0.002 | 0.002 |
| 2, 5-Dimethyl-4-hydroxy-3 (2H)-furanone | 0.3 | 0.3 |
| Ethanol | Residue | Residue |
| Total | 1000.0 | 1000.0 |

Each of the pineapple flavor compositions (29) and (30) prepared according to the above formulation was added to water at a concentration of 0.1%, and a sensory test was conducted by 11 expert panelists. As a result, all of the panelists commented that the flavor composition (29) was significantly superior to the flavor composition (30) in terms of impartation of a natural fruit juice feeling. The same was true for the case where no matter which of the four kinds of isomers 6a,7-dimethylhexahydro-2H-2,5-methanocyclopenta [b] furan in the flavor composition (29) was, it was replaced with an isomeric mixture.

### (Example 18) Production of Banana Flavor Composition

A banana flavor composition (31) was prepared based on the following formulation using 6a,7-dimethylhexahydro-2H-2,5-methanocyclopenta [b] furan produced in Example 1. For comparison, a banana flavor composition (32) was prepared based on the following formulation.

**[Table 18]**

| (Component) | Flavor composition (31) (parts by weight) | Flavor composition (32) (parts by weight) |
|---|---|---|
| 6a,7-dimethylhexahydro-2H-2,5-methanocyclopenta [b] furan in Example 1 | 0.1 | - |
| Isoamyl acetate | 50.0 | 50.0 |
| Isoamyl butyrate | 40.0 | 40.0 |
| Ethyl butyrate | 20.0 | 20.0 |
| Cis-3-hexenol | 5.0 | 5.0 |
| Eugenol | 0.05 | 0.05 |
| Ethanol | Residue | Residue |
| Total | 1000.0 | 1000.0 |

Each of the banana flavor compositions (31) and (32) prepared according to the above formulation was added to water at a concentration of 0.1%, and a sensory test was conducted by 11 expert panelists. As a result, all of the panelists commented that the flavor composition (31) was significantly superior to the flavor composition (32) in terms of impartation of a natural fruit juice feeling. The same was true for the case where no matter which of the four kinds of isomers 6a,7-dimethylhexahydro-2H-2,5-methanocyclopenta [b] furan in the flavor composition (31) was, it was replaced with an isomeric mixture.

### (Example 19) Production of strawberry Flavor Composition

A strawberry flavor composition (33) was prepared based on the following formulation using 6a,7-dimethylhexahydro-2H-2,5-methanocyclopenta [b] furan produced in Example 1. For comparison, a strawberry flavor composition (34) was prepared based on the following formulation.

**[Table 19]**

| (Component) | Flavor composition (33) (parts by weight) | Flavor composition (34) (parts by weight) |
|---|---|---|
| 6a,7-dimethylhexahydro-2H-2,5-methanocyclopenta [b] furan in Example 1 | 0.1 | - |
| α-Ionone | 0.1 | 0.1 |
| Ethyl butyrate | 90.0 | 90.0 |
| Ethyl isovalerate | 20.0 | 20.0 |
| Ethyl hexanoate | 1.0 | 1.0 |
| Ethyl 3-methyl-3-phenylglycidate | 10.0 | 10.0 |
| Cis-3-hexenol | 70.0 | 70.0 |
| Nonanoic acid | 3.0 | 3.0 |
| Linalool | 0.4 | 0.4 |
| Ethyl maltol | 100.0 | 100.0 |
| Vanillin | 0.4 | 0.4 |
| γ-undecalactone | 2.0 | 2.0 |
| γ-dodecalactone | 3.0 | 3.0 |
| Methyl heptyne carbonate | 0.5 | 0.5 |
| Damascenone | 0.002 | 0.002 |
| Propylene glycol (solvent) | Residue | Residue |
| Total | 1000.0 | 1000.0 |

Each of the strawberry flavor compositions (33) and (34) prepared according to the above formulation was added to water at a concentration of 0.1%, and a sensory test was conducted by 11 expert panelists. As a result, all of the panelists commented that the flavor composition (33) was significantly superior to the flavor composition (34) in terms of impartation of a natural fruit juice feeling. The same was true for the case where no matter which of the four kinds of isomers 6a,7-dimethylhexahydro-2H-2,5-methanocyclopenta [b] furan in the flavor composition (33) was, it was replaced with an isomeric mixture.

### (Example 20) Production of Raspberry Flavor Composition

A raspberry flavor composition (35) was prepared based on the following formulation using 6a,7-dimethylhexahydro-2H-2,5-methanocyclopenta [b] furan produced in Example 1. For comparison, a raspberry flavor composition (36) was prepared based on the following formulation.

**[Table 20]**

| (Component) | Flavor composition (35) (parts by weight) | Flavor composition (36) (parts by weight) |
|---|---|---|
| 6a,7-dimethylhexahydro-2H-2,5-methanocyclopenta [b] furan in Example 1 | 0.1 | - |
| α-Ionone | 15.0 | 15.0 |
| Propyl acetate | 200.0 | 200.0 |
| Ethyl butyrate | 90.0 | 90.0 |
| Isoamyl butyrate | 3.0 | 3.0 |
| Trans-2-hexenyl acetate | 30.0 | 30.0 |
| Cis-3-hexenol | 60.0 | 60.0 |
| Trans-2-hexenal | 30.0 | 30.0 |
| Acetic acid | 200.0 | 200.0 |
| 2-Methylbutyrate | 70.0 | 70.0 |
| Linalool | 10.0 | 10.0 |
| γ-decalactone | 5.0 | 5.0 |
| 2-Phenylethyl alcohol | 2.0 | 2.0 |
| Raspberry ketone | 150.0 | 150.0 |
| Damascenone | 10.0 | 10.0 |
| Dimethyl sulfide | 0.2 | 0.2 |
| Propylene glycol (solvent) | Residue | Residue |
| Total | 1000.0 | 1000.0 |

Each of the raspberry flavor compositions (35) and (36) prepared according to the above formulation was added to water at a concentration of 0.1%, and a sensory test was conducted by 11 expert panelists. As a result, all of the panelists commented that the flavor composition (35) was significantly superior to the flavor composition (36) in terms of impartation of a natural fruit juice feeling. The same was true for the case where no matter which of the four kinds of isomers 6a,7-dimethylhexahydro-2H-2,5-methanocyclopenta [b] furan in the flavor composition (35) was, it was replaced with an isomeric mixture.

### (Example 21) Production of Blueberry Flavor Composition

A blueberry flavor composition (37) was prepared based on the following formulation using 6a,7-dimethylhexahydro-2H-2,5-methanocyclopenta [b] furan produced in Example 1. For comparison, a blueberry flavor composition (38) was prepared based on the following formulation.

**[Table 21]**

| (Component) | Flavor composition (37) (parts by weight) | Flavor composition (38) (parts by weight) |
|---|---|---|
| 6a,7-dimethylhexahydro-2H-2,5-methanocyclopenta [b] furan in Example 1 | 0.1 | - |
| α-Ionone | 2.0 | 2.0 |
| Isobutyl acetate | 10.0 | 10.0 |
| Isoamyl acetate | 70.0 | 70.0 |
| Ethyl butyrate | 300.0 | 300.0 |
| Dimethyl benzyl carbinyl butyrate | 8.0 | 8.0 |
| Cis-3-hexenyl acetate | 1.0 | 1.0 |
| Cis-3-hexenol | 6.0 | 6.0 |
| Trans-2-hexenal | 3.0 | 3.0 |
| Acetic acid | 300.0 | 300.0 |
| Linalool | 1.0 | 1.0 |
| Geraniol | 0.2 | 0.2 |
| Ethyl Maltol | 10.0 | 10.0 |
| γ-undecalactone | 3.0 | 3.0 |
| 2-Phenylethyl alcohol | 2.0 | 2.0 |
| Raspberry ketone | 100.0 | 100.0 |
| β-Damascone | 1.0 | 1.0 |
| Dimethyl sulfide | 1.0 | 1.0 |
| Propylene glycol (solvent) | Residue | Residue |
| Total | 1000.0 | 1000.0 |

Each of the blueberry flavor compositions (37) and (38) prepared according to the above formulation was added to water at a concentration of 0.1%, and a sensory test was conducted by 11 expert panelists. As a result, all of the panelists commented that the flavor composition (37) was significantly superior to the flavor composition (38) in terms of impartation of a natural fruit juice feeling. The same was true for the case where no matter which of the four kinds of isomers 6a,7-dimethylhexahydro-2H-2,5-methanocyclopenta [b] furan in the flavor composition (37) was, it was replaced with an isomeric mixture.

### (Example 22) Production of Sparkling Wine Flavor Composition

A sparkling wine flavor composition (39) was prepared based on the following formulation using 6a,7-dimethylhexahydro-2H-2,5-methanocyclopenta [b] furan produced in Example 1. For comparison, a sparkling wine flavor composition (40) was prepared based on the following formulation.

**[Table 22]**

| (Component) | Flavor composition (39) (parts by weight) | Flavor composition (40) (parts by weight) |
|---|---|---|
| 6a,7-dimethylhexahydro-2H-2,5-methanocyclopenta [b] furan in Example 1 | 0.01 | - |
| Isoamyl acetate | 3.0 | 3.0 |
| Ethyl hexanoate | 1.0 | 1.0 |
| Ethyl octanoate | 2.0 | 2.0 |
| Ethyl decanoate | 0.6 | 0.6 |
| 2-Phenylethyl alcohol | 1.0 | 1.0 |
| Octanoic acid | 1.0 | 1.0 |
| Decanoic acid | 0.8 | 0.8 |
| Damascenone | 0.005 | 0.005 |
| Ethyl 2-methylbutyrate | 0.05 | 0.05 |
| Ethyl butyrate | 0.3 | 0.3 |
| Geraniol | 0.02 | 0.02 |
| Linalool | 0.03 | 0.03 |
| 2-Phenylethyl acetate | 0.5 | 0.5 |
| Ethyl acetate | 35.0 | 35.0 |
| Isoamyl alcohol | 40.0 | 40.0 |
| Dimethyl sulfide | 0.03 | 0.03 |
| Ethanol | Residue | Residue |
| Total | 1000.0 | 1000.0 |

Each of the sparkling wine flavor compositions (39) and (40) prepared according to the above formulation was added to water at a concentration of 0.1%, and a sensory test was conducted by 11 expert panelists. As a result, all of the panelists commented that the flavor composition (39) was significantly superior to the flavor composition (40) in terms of impartation of a light alcoholic feeling with a sparkling feeling. The same was true for the case where no matter which of the four kinds of isomers 6a,7-dimethylhexahydro-2H-2,5-methanocyclopenta [b] furan in the flavor composition (39) was, it was replaced with an isomeric mixture.

### (Example 23) Production of Beer Flavor Composition

A beer flavor composition (41) was prepared based on the following formulation using 6a,7-dimethylhexahydro-2H-2,5-methanocyclopenta [b] furan produced in Example 1. For comparison, a beer flavor composition (42) was prepared based on the following formulation.

**[Table 23]**

| (Component) | Flavor composition (41) (parts by weight) | Flavor composition (42) (parts by weight) |
|---|---|---|
| 6a,7-dimethylhexahydro-2H-2,5-methanocyclopenta [b] furan in Example 1 | 0.01 | - |
| Ethyl acetate | 50.0 | 50.0 |
| Isoamyl alcohol | 50.0 | 50.0 |
| 2-Phenylethyl alcohol | 20.0 | 20.0 |
| Octanoic acid | 8.0 | 8.0 |
| Hexanoic acid | 3.0 | 3.0 |
| Decanoic acid | 0.5 | 0.5 |
| Isoamyl acetate | 2.0 | 2.0 |
| 2-Phenylethyl acetate | 2.0 | 2.0 |
| Methionol | 1.0 | 1.0 |
| Ethyl octanoate | 0.5 | 0.5 |
| Ethyl hexanoate | 0.1 | 0.1 |
| Ethyl decanoate | 0.05 | 0.05 |
| 4-Vinylguaiacol | 0.2 | 0.2 |
| γ-nonalactone | 0.02 | 0.02 |
| Linalool | 0.01 | 0.01 |
| Sotolon | 0.002 | 0.002 |
| Damascenone | 0.001 | 0.001 |
| Propylene glycol (solvent) | Residue | Residue |
| Total | 1000.0 | 1000.0 |

Each of the beer flavor compositions (41) and (42) prepared according to the above formulation was added to water at a concentration of 0.1%, and a sensory test was conducted by 11 expert panelists. As a result, all of the panelists commented that the flavor composition (41) was significantly superior to the flavor composition (42) in terms of impartation of a light alcoholic feeling with a foaming feeling. The same was true for the case where no matter which of the four kinds of isomers 6a,7-dimethylhexahydro-2H-2,5-methanocyclopenta [b] furan in the flavor composition (41) was, it was replaced with an isomeric mixture.

### (Example 24) Production of Green Tea Flavor Composition

A green tea flavor composition (43) was prepared based on the following formulation using 6a,7-dimethylhexahydro-2H-2,5-methanocyclopenta [b] furan produced in Example 1. For comparison, a green tea flavor composition (44) was prepared based on the following formulation.

**[Table 24]**

| (Component) | Flavor composition (43) (parts by weight) | Flavor composition (44) (parts by weight) |
|---|---|---|
| 6a,7-dimethylhexahydro-2H-2,5-methanocyclopenta [b] furan in Example 1 | 0.01 | - |
| α-Ionone | 0.1 | 0.1 |
| Cis-3-hexenyl hexanoate | 5.0 | 5.0 |
| Methyl jasmonate | 3.0 | 3.0 |
| Hexanal | 0.3 | 0.3 |
| Hexanol | 2.0 | 2.0 |
| Cis-3-hexenol | 1.0 | 1.0 |
| α-terpineol | 10.0 | 10.0 |
| Linalool | 1.0 | 1.0 |
| Nerol | 2.0 | 2.0 |
| Phenylacetaldehyde | 2.0 | 2.0 |
| Cis-Jasmone | 8.0 | 8.0 |
| β-Ionone | 1.0 | 1.0 |
| Indole | 0.6 | 0.6 |
| Dimethyl sulfide | 5.0 | 5.0 |
| Propylene glycol (solvent) | Residue | Residue |
| Total | 1000.0 | 1000.0 |

Each of the green tea flavor compositions (43) and (44) prepared according to the above formulation was added to water at a concentration of 0.1%, and a sensory test was conducted by 11 expert panelists. As a result, all of the panelists commented that the flavor composition (43) was significantly superior to the flavor composition (44) in terms of impartation of spreading natural sweet aroma. The same was true for the case where no matter which of the four kinds of isomers 6a,7-dimethylhexahydro-2H-2,5-methanocyclopenta [b] furan in the flavor composition (43) was, it was replaced with an isomeric mixture.

### (Example 25) Production of Black Tea Flavor Composition

A black tea flavor composition (45) was prepared based on the following formulation using 6a,7-dimethylhexahydro-2H-2,5-methanocyclopenta [b] furan produced in Example 1. For comparison, a black tea flavor composition (46) was prepared based on the following formulation.

**[Table 25]**

| (Component) | Flavor composition (45) (parts by weight) | Flavor composition (46) (parts by weight) |
|---|---|---|
| 6a,7-dimethylhexahydro-2H-2,5-methanocyclopenta [b] furan in Example 1 | 0.01 | - |
| 2-Phenylethyl alcohol | 1.0 | 1.0 |
| Geraniol | 1.0 | 1.0 |
| Linalool oxide | 0.5 | 0.5 |
| Hexanal | 0.05 | 0.05 |
| Phenyl acetal dehyde | 0.1 | 0.1 |
| Cis-3-hexenol | 1.0 | 1.0 |
| β-Ionone | 0.001 | 0.001 |
| Linalool | 1.0 | 1.0 |
| Damascenone | 0.03 | 0.03 |
| Isoeugenol | 0.001 | 0.001 |
| Propylene glycol (solvent) | Residue | Residue |
| Total | 1000.0 | 1000.0 |

Each of the black tea flavor compositions (45) and (46) prepared according to the above formulation was added to water at a concentration of 0.1%, and a sensory test was conducted by 11 expert panelists. As a result, all of the panelists commented that the flavor composition (45) was significantly superior to the flavor composition (46) in terms of impartation of spreading natural sweet aroma. The same was true for the case where no matter which of the four kinds of isomers 6a,7-dimethylhexahydro-2H-2,5-methanocyclopenta [b] furan in the flavor composition (45) was, it was replaced with an isomeric mixture.

### (Example 26) Addition to Commercially Available Orange Juice Beverage

6a,7-dimethylhexahydro-2H-2,5-methanocyclopenta [b] furan produced in Example 1 was added to a commercially available orange juice beverage at a concentration of 0.01 ppm, and a sensory test was conducted by 11 expert panelists. As a result, all of the panelists commented that a real fruit juice feeling, freshness, and fresh feeling were imparted, which were significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 27) Addition to Commercially Available Lemon Juice Beverage

6a,7-dimethylhexahydro-2H-2,5-methanocyclopenta [b] furan produced in Example 1 was added to a commercially available lemon juice beverage at a concentration of 0.01 ppm, and a sensory test was conducted by 11 expert panelists. As a result, all of the panelists commented that fresh feeling and fresh and sweet aroma were imparted, which were significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 28) Addition to Commercially Available Grapefruit Juice Beverage

6a,7-dimethylhexahydro-2H-2,5-methanocyclopenta [b] furan produced in Example 1 was added to a commercially available grapefruit juice beverage at a concentration of 0.01 ppm, and a sensory test was conducted by 11 expert panelists. As a result, all of the panelists commented that a real fruit juice feeling, freshness, and fresh feeling were imparted, which were significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 29) Addition to Commercially Available Lime Juice Beverage

6a,7-dimethylhexahydro-2H-2,5-methanocyclopenta [b] furan produced in Example 1 was added to a commercially available lime juice beverage at a concentration of 0.01 ppm, and a sensory test was conducted by 11 expert panelists. As a result, all of the panelists commented that fresh and sweet aroma was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 30) Addition to Commercially Available Yuzu Juice Beverage

6a,7-dimethylhexahydro-2H-2,5-methanocyclopenta [b] furan produced in Example 1 was added to a commercially available yuzu juice beverage at a concentration of 0.01 ppm, and a sensory test was conducted by 11 expert panelists. As a result, all of the panelists commented that fresh and sweet aroma was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 31) Addition to Commercially Available Cider Beverage

6a,7-dimethylhexahydro-2H-2,5-methanocyclopenta [b] furan produced in Example 1 was added to a commercially available cider beverage at a concentration of 0.1 ppm, and a sensory test was conducted by 11 expert panelists. As a result, all of the panelists commented that citrus-like fresh sweetness and a carbonation feeling was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 32) Addition to Commercially Available Apple Juice Beverage

6a,7-dimethylhexahydro-2H-2,5-methanocyclopenta [b] furan produced in Example 1 was added to a commercially available apple juice beverage at a concentration of 0.1 ppm, and a sensory test was conducted by 11 expert panelists. As a result, all of the panelists commented that a natural fruit juice feeling was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 33) Addition to Commercially Available Grape Juice Beverage

6a,7-dimethylhexahydro-2H-2,5-methanocyclopenta [b] furan produced in Example 1 was added to a commercially available grape juice beverage at a concentration of 0.1 ppm, and a sensory test was conducted by 11 expert panelists. As a result, all of the panelists commented that a natural fruit juice feeling was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 34) Addition to Commercially Available Peach Juice Beverage

6a,7-dimethylhexahydro-2H-2,5-methanocyclopenta [b] furan produced in Example 1 was added to a commercially available peach juice beverage at a concentration of 0.1 ppm, and a sensory test was conducted by 11 expert panelists. As a result, all of the panelists commented that a natural fruit juice feeling was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 35) Addition to Commercially Available Sparkling Wine Taste Beverage

6a,7-dimethylhexahydro-2H-2,5-methanocyclopenta [b] furan produced in Example 1 was added to a commercially available sparkling wine taste beverage at a concentration of 0.01 ppm, and a sensory test was conducted by 11 expert panelists. As a result, all of the panelists commented that a light alcoholic feeling with a sparkling feeling was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 36) Addition to Commercially Available Beer Taste Beverage

6a,7-dimethylhexahydro-2H-2,5-methanocyclopenta [b] furan produced in Example 1 was added to a commercially available beer taste beverage at a concentration of 0.01 ppm, and a sensory test was conducted by 11 expert panelists. As a result, all of the panelists commented that a light alcoholic feeling with a foaming feeling was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 37) Addition to Commercially Available Green Tea Beverage

6a,7-dimethylhexahydro-2H-2,5-methanocyclopenta [b] furan produced in Example 1 was added to a commercially available green tea beverage at a concentration of 0.01 ppm, and a sensory test was conducted by 11 expert panelists. As a result, all of the panelists commented that spreading natural sweet aroma was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 38) Addition to Commercially Available Black Tea

6a,7-dimethylhexahydro-2H-2,5-methanocyclopenta [b] furan produced in Example 1 was added to a commercially available black tea beverage at a concentration of 0.01 ppm, and a sensory test was conducted by 11 expert panelists. As a result, all of the panelists commented that spreading natural sweet aroma was imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 39) Toothpaste Flavoring Evaluation

A grapefruit-like flavor composition (47) for a toothpaste was prepared based on the following formulation using 6a,7-dimethylhexahydro-2H-2,5-methanocyclopenta [b] furan produced in Example 1. For comparison, a grapefruit-like flavor composition (48) for a toothpaste was prepared based on the following formulation.

**[Table 26]**

| (Component) | Flavor composition (47) (parts by weight) | Flavor composition (48) (parts by weight) |
|---|---|---|
| 6a,7-dimethylhexahydro-2H-2,5-methanocyclopenta [b] furan in Example 1 | 0.02 | - |
| L-Menthol | 400.0 | 400.0 |
| Peppermint oil | 150.0 | 150.0 |
| Grapefruit oil (cold pressed) | 100.0 | 100.0 |
| Mint oil | 100.0 | 100.0 |
| Anethole | 60.0 | 60.0 |
| Eucalyptol | 20.0 | 20.0 |
| Ethanol | Residue | Residue |
| Total | 1000.0 | 1000.0 |

The formulation of toothpaste base materials is as follows.

**[Table 27]**

| (Component) | (Parts by weight) |
|---|---|
| Calcium carbonate | 400.0 |
| Anhydrous silicic acid | 16.5 |
| Sorbitol (70%) | 240.0 |
| Sodium lauryl sulfate | 13.0 |
| Carboxymethyl cellulose sodium salt | 12.5 |
| Carrageenan | 3.0 |
| Sodium Benzoate | 4.0 |
| Sodium saccharin | 1.5 |
| Purified water | 259.5 |
| Propylene glycol | 40.0 |
| Total | 990.0 |

The following toothpastes (49) and (50) were prepared using the flavor composition and the toothpaste base materials which were prepared according to the above formulation, and subjected to sensory evaluation.
(49) Toothpaste base material 990 g + flavor composition (47) 10 g
(50) Toothpaste base material 990 g + flavor composition (48) 10 g

A sensory test was conducted on these toothpastes by 11 expert panelists, and as a result, all of the panelists commented that the toothpaste (49) using the flavor composition (47) was significantly superior to the toothpaste (50) using the flavor composition (48) in terms of impartation of a real fruit juice feeling, freshness, and fresh feeling. The same was true for the case where no matter which of the four kinds of isomers 6a,7-dimethylhexahydro-2H-2,5-methanocyclopenta [b] furan in the flavor composition (47) was, it was replaced with an isomeric mixture.

### (Example 40) Addition to High Sweetness Sweetener (Rebaudioside A) Aqueous Solution

6a,7-dimethylhexahydro-2H-2,5-methanocyclopenta [b] furan produced in Example 1 was added to a 0.028 mass% of rebaudioside A aqueous solution at a concentration of 0.0001 ppm, and a sensory test was conducted by 11 expert panelists. As a result, all of the panelists commented that an unpleasant flavor diminished, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 41) Addition to High Sweetness Sweetener (Aspartame) Aqueous Solution

6a,7-dimethylhexahydro-2H-2,5-methanocyclopenta [b] furan produced in Example 1 was added to a 0.04 mass% of aspartame aqueous solution at a concentration of 0.0001 ppm, and a sensory test was conducted by 11 expert panelists. As a result, all of the panelists commented that a bulge was imparted in the second half, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 42) Addition to High Sweetness Sweetener (Sucralose) Aqueous Solution

6a,7-dimethylhexahydro-2H-2,5-methanocyclopenta [b] furan produced in Example 1 was added to a 0.013 mass% of sucralose aqueous solution at a concentration of 0.0001 ppm, and a sensory test was conducted by 11 expert panelists. As a result, all of the panelists commented that a taste thickness and sweetness bulge were imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

### (Example 43) Addition to High Sweetness Sweetener (Acesulfame K) Aqueous Solution

6a,7-dimethylhexahydro-2H-2,5-methanocyclopenta [b] furan produced in Example 1 was added to a 0.04 mass% of acesulfame K aqueous solution at a concentration of 0.0001 ppm, and a sensory test was conducted by 11 expert panelists. As a result, all of the panelists commented that a taste thickness and sweetness bulge were imparted, which was significantly excellent, since 6a,7-dimethylhexahydro-2H-2,5-methanocyclopenta [b] furan was added. The same was true for the case where no matter which of the four kinds of isomers 6a,7-dimethylhexahydro-2H-2,5-methanocyclopenta [b] furan was, it was replaced with an isomeric mixture.

While the present invention has been described in detail with reference to specific embodiments, it will be apparent to those skilled in the art that various changes and modifications can be made without departing from the spirit and scope of the present invention. The present application is based on a Japanese Patent Application (Japanese Patent Application No. 2019-133854) filed on July 19, 2019, contents of which are incorporated herein by reference.

### INDUSTRIAL APPLICABILITY

According to the flavor composition of the present invention, it is possible to impart or enhance a citrus-like aroma and flavor to various products such as foods or beverages, oral compositions, tobacco products, and consumer products.

## Claims

1. A flavor composition comprising 6a,7-dimethylhexahydro-2H-2,5-methanocyclopenta [b] furan.

2. The flavor composition according to claim 1, which is used for foods or beverages.

3. A compound represented by the following formula (1).

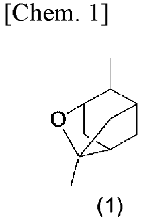

4. A food or beverage, oral composition, tobacco product, or consumer product, comprising the flavor composition according to claim 1 or 2 or the compound according to claim 3.

5. A method for producing a food or beverage, oral composition, tobacco product, or consumer product, the method comprising adding the flavor composition according to claim 1 or 2 or the compound according to claim 3.
